# NEUE EUROPÄISCHE PATENTSCHRIFT

(11) **EP 0 554 711 B2**
(45) Veröffentlichungstag und Bekanntmachung des Hinweises auf die Entscheidung über den Einspruch: **19.07.2000**
(45) Hinweis auf die Patenterteilung: 13.11.1996
(21) Anmeldenummer: 93100753.8
(22) Anmeldetag: 20.01.1993
(51) Int. Cl.: A61B 19/00, A61B 6/00, B25J 19/00

(54) **Motorisches Stativ**
Actuated support
Support motorisé

(30) Priorität: 01.02.1992 DE 4202922
(43) Veröffentlichungstag der Anmeldung: 11.08.1993
(73) Patentinhaber: Firma Carl Zeiss, D-73446 Oberkochen (DE); CARL ZEISS-STIFTUNG HANDELND ALS CARL ZEISS, D-89518 Heidenheim (Brenz) (DE)
(72) Erfinder: Schweizer, Jürgen, Dr., W-7084 Westerhofen (DE); Gärtner, Hartmut, W-7082 Oberkochen (DE); Luber, Joachim, W-7087 Essingen-Forst (DE)

(56) Entgegenhaltungen:
- EP-A- 0 066 917
- EP-A- 0 386 842
- DE-A- 2 624 378
- DE-A- 3 026 932
- DE-A- 3 416 823
- DE-A- 4 032 207
- DE-C- 3 135 608
- US-A- 3 770 955
- US-A- 4 684 088
- US-A- 4 753 128
- US-A- 4 828 451
- US-A- 5 078 140

## Beschreibung

Gegenstand der Erfindung ist ein motorisches Stativ gemäß dem Oberbegriff des Anspruches 1, das aus dem Dokument DE-A-40 32 207 bekannt ist.

Herkömmliche Stative im medizinischen Einsatz bieten die Möglichkeit, diverse Diagnose- oder Therapieinstrumente in bis zu sechs Freiheitsgraden manuell zu positionieren. Ein derartiges manuelles Stativ wird z.B. in der EP-PS 0 023 003 beschrieben. Aufgrund des manuellen Betriebes resultieren jedoch eine Reihe von Nachteilen für den Chirurgen. So erfordern unbeabsichtigte Fehlbewegungen ein zeitaufwendiges und umständliches Nachkorrigieren. Als negativ erweist sich bei mechanisch ausbalancierten Systemen weiterhin, daß diese jeweils nur für ein definiertes Instrumentengewicht exakt ausbalanciert sind. Dies bedeutet, daß eine Änderung des Instrumentengewichtes ein erneutes Ausbalancieren des Stativsystems mit einer Genauigkeit von etwa 100 g erfordert. Während einer Operation ist ein derartiges erneutes Ausbalancieren nach einem eventuellen Instrumentenwechsel extrem störend und umständlich. Weiterhin erfordert selbst bei gut ausbalancierten mechanischen Stativsystemen das Beschleunigen bzw. Abbremsen der Instrumentenbewegung eine gewisse beschleunigungsproportionale Bedienkraft, was bei mikrochirurgischen Arbeiten ebenfalls störend wirkt. Schließlich ist mit manuell bewegten Stativen im allgemeinen auch kein stereotaktisches Arbeiten möglich, da exakte Lageinformationen über das momentane Arbeitsfeld üblicherweise nicht erfaßt werden.

Bekannt ist aus der DE-OS 40 32 207 weiterhin ein Operationsmikroskop, das an einem motorisch angetriebenen Mehrgelenk-Mechanismus angeordnet ist. Mittels geeigneter Weg- und Winkeldetektoren ist somit ein eingeschränkter stereotaktischer Einsatz des Operationsmikroskopes möglich. Als nachteilig bei diesem System ist jedoch anzusehen, daß keine Sicherungsmaßnahmen beim eventuellen Ausfall der Antriebselektrik bzw. der damit gekoppelten Weg- und Winkeldetektoren vorgesehen sind. So weist der dargestellte Mehrgelenk-Mechanismus keinerlei Massenausgleich auf, der das System beim Ausfall der Antriebseinheiten stabilisieren könnte und ein zumindest eingeschränktes weiteres Verfahren ermöglichen würde. Zudem ist bei einem möglichen Versagen der Antriebe auch ein Ausfall der mit den Antrieben gekoppelten Weg- bzw. Winkeldetektoren die Folge, d.h. unter Umständen resultiert daraus ein kompletter Systemausfall während einer Operation.

Aus der US 4,753,128 ist desweiteren ein Industrie-Roboter bekannt, bei dem zum Ausgleich von gewichtsabhängigen Drehmomenten ein Feder-Kompensationsmechanismus vorgeschlagen wird. Zur Problematik der oben angesprochenen Ausfall-Sicherheit medizinischer Stative liefert dieses Dokument jedoch keine weiteren Hinweise.

Aufgabe der vorliegenden Erfindung ist es daher, ein motorisches Stativ für den medizinischen Einsatz zu schaffen, das den stereotaktischen Einsatz verschiedenster Diagnose- oder Therapieinstrumente ermöglicht. Dabei soll eine höchstmögliche Sicherheit und Positionierpräzision sowohl während des normalen Betriebes als auch bei einem eventuellen Ausfall der Antriebseinheiten gewährleistet sein. Insbesondere soll in diesem Fall der manuelle Betrieb eines derartigen Statives noch möglich sein.

Diese Aufgabe wird gelöst durch ein motorisches Stativ mit den Merkmalen des Anspruchs 1. Vorteilhafte Ausgestaltungen sind Gegenstand der folgenden Unteransprüche.

Ein mechanisch grob ausbalanciertes Stativ, bestehend aus mehreren Stativgliedern, Tragarmen bzw. Tragsäulen, wird mit speziellen Antriebseinheiten versehen, die ein definiertes Positionieren verschiedenster medizinischer Diagnose- oder Therapieinstrumente erlauben. Eine Steuereinheit erfaßt hierzu laufend die aktuellen Winkelmeßwerte der in den einzelnen Antriebseinheiten angeordneten Winkelgebern und bestimmt daraus die aktuellen Raumkoordinaten des jeweils verwendeten Instrumentes. Über entsprechende Steuersignale der Steuereinheit an die einzelnen Antriebseinheiten ist ein definiertes Positionieren des Instrumentes möglich. Dabei gibt die Steuereinheit an jede Antriebseinheit bestimmte, anzufahrende Sollwerte vor, die dann von jeder Antriebseinheit in separaten Regelkreisen angefahren werden. Somit wird ein stereotaktischer Einsatz verschiedenster medizinischer Diagnose- oder Therapieinstrumente realisierbar. Die Steuereinheit weist des weiteren eine Eingabeschnittstelle auf, die eine Eingabe bestimmter Zielkoordinaten für das verwendete Instrument ermöglicht, welche vom erfindungsgemäßen motorischen Stativ anschließend angefahren werden, bzw. das verwendete Instrument mit Hilfe des erfindungsgemäßen motorischen Statives an die gewünschte Raumposition positioniert wird. Eine derartige Eingabeschnittstelle kann hierbei in verschiedensten Ausführungsformen in Kombination mit dem motorischen Stativ eingesetzt werden. In Frage kommen z.B. diverse berührungsempfindliche Sensoren, die vom Chirurgen bedient werden oder aber optische Systeme bzw. eine Eingabetastatur, welche jeweils zur Vorgabe bestimmter Zielkoordinaten für das erfindungsgemäße motorische Stativ dienen.

Die mechanische Ausbalancierung des Gesamtsystems erfolgt in einer bevorzugten Ausführungsform über ein Gelenkparallelogramm mit Ausgleichsgewichten sowie die erfindungsgemäße konstruktive Ausgestaltung bestimmter Tragarme bzw. die entsprechende Anordnung von Ausgleichgewichten. Alternativ wäre auch eine Ausbalancierung mit Federn realisierbar. Damit ist gewährleistet, daß bei einem eventuellen Ausfall der Antriebseinheiten während einer Operation das Stativ weiterhin zumindest beschränkt einsatzfähig bleibt und manuell bewegt werden kann. Die mechanische Ausbalancierung des Gesamtsystems kann hierbei mit einer Genauigkeit im kg-Bereich erfolgen. Aus dem erfindungsgemäßen Aufbau der einzelnen Antriebseinheiten, insbesondere der Dimensionierung der Getriebereibung sowie der Verwendung je zweier Winkelgeber pro Antriebseinheit resultieren weitere wichtige Vorteile des erfindungsgemäßen motorischen Statives im Hinblick auf die Einsatzsicherheit. So ist die Getriebereibung der einzelnen Antriebseinheiten derart aufeinander abgestimmt, daß ein Spielraum für das Gewicht des jeweils verwendeten Instrumentes besteht und die mechanische Ausbalancierung auch bei Variation des Instrumentengewichtes noch gegeben ist. Bei der Umrüstung auf ein anderes medizinisches Therapie- oder Diagnoseinstrument während einer Operation ist somit keine zusätzliche weitere mechanische Ausbalancierung nötig, die umständlich bzw. zeitaufwendig wäre. Innerhalb eines definierten Gewichtsintervalls können demzufolge verschiedenste medizinische Therapie- oder Diagnoseinstrumente in Verbindung mit dem erfindungsgemäßen motorischen Stativ eingesetzt werden. Weitere sicherheitsrelevante Vorteile resultieren aus der erfindungsgemäßen Verwendung von je zwei Winkelgebern pro Antriebseinheit, von denen einer mit dem jeweiligen Antriebsmotor gekoppelt ist, während der zweite unabhängig von diesem Antriebsmotor arbeitet und direkt die jeweilige Gelenkbewegung registriert. Neben der laufenden gegenseitigen Meßwertkontrolle über die Steuereinheit während des üblichen motorischen Betriebes und der daraus resultierenden höheren Positionierungsgenauigkeit, ist dadurch weiterhin gewährleistet, daß bei Ausfall des Antriebsmotors eine Meßwerterfassung über den zweiten unabhängigen Winkelgeber möglich ist. Eine Kenntnis der aktuellen Instrumentenposition ist somit auch in diesem Fall gewährleistet. Desweiteren ist beim Einsatz eines speziellen Absolut-Winkelgebers sichergestellt, daß auch ohne eventuelle Referenzmessungen immer die aktuellen Winkelmeßwerte bekannt sind.

Die erwähnte Dimensionierung der Getriebereibung bietet weiterhin den Vorteil, daß auch bei eventueller schiefer Aufstellung des erfindungsgemäßen motorischen Statives ein noch ausbalanciertes System zur Verfügung steht und sich nicht selbständig unkontrolliert bewegt.

Das Fußteil weist des weiteren ein- und ausfahrbare Rollen auf, die ein leichtes Verfahren des gesamten motorischen Statives ermöglichen. In einer bevorzugten Ausführungsform können diese Rollen motorisch ein- und ausgefahren werden.

Insbesondere geeignet ist ein derartiges motorisches Stativ zur Verwendung in Verbindung mit einem Operationsmikroskop innerhalb der stereotaktischen Mikrochirurgie. Jedoch ist auch der stereotaktische Einsatz verschiedener anderer chirurgischer Instrumente in Kombination mit dem erfindungsgemäßen motorischen Stativ möglich. In Frage kommen z.B. Endoskope, intra-operative Sonden, Biopsie-Kanülen, chirurgische Bohrer oder ähnliche Instrumente.

Weitere Einzelheiten sowie Vorteile des erfindungsgemäßen motorischen Statives ergeben sich aus der Beschreibung eines Ausführungsbeispieles anhand der beigefügten Zeichnungen.

Dabei zeigt
- Fig. 1: eine schematische Darstellung eines möglichen Aufbaus des erfindungsgemäßen motorischen Statives;
- Fig. 2: ein Ausführungsbeispiel des erfindungsgemäßen motorischen Statives;
- Fig. 3: den Aufbau einer Antriebseinheit des erfindungsgemäßen motorischen Statives.

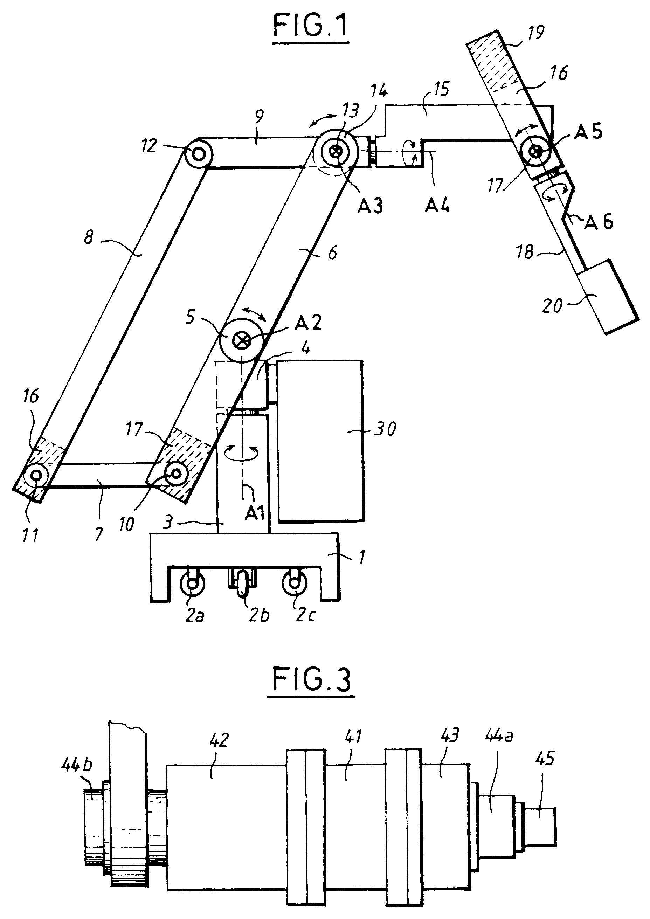

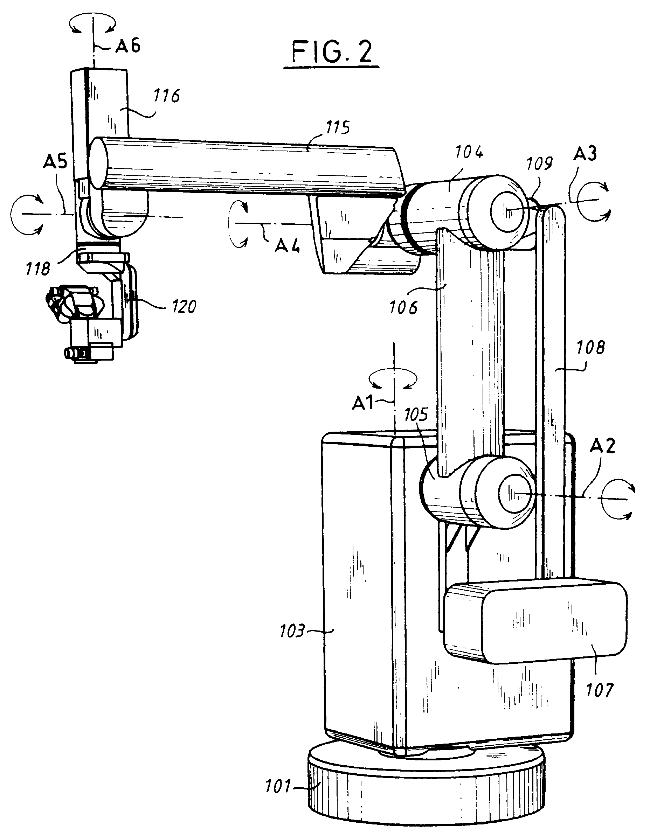

In Fig. 1 ist ein möglicher Aufbau des erfindungsgemäßen motorischen Statives schematisch dargestellt, wobei die Ausbalancierung mit Ausgleichsgewichten realisiert wird. Das erfindungsgemäße motorische Stativ ist auf einem Fußteil (1) angeordnet, das an seiner Unterseite motorisch ein- und ausfahrbare Rollen (2a, 2b, 2c) besitzt, so daß ein leichtes Verfahren des gesamten motorischen Statives möglich ist. Während des eigentlichen Operationsbetriebes sind diese Rollen (2a, 2b, 2c) eingezogen, um einen sicheren Stand des Gesamtsystems zu gewährleisten. Auf dem Fußteil (1 ist eine vertikale Tragsäule (3) angeordnet, auf der wiederum ein erstes Stativglied (4) um eine vertikale Achse A1 über ein erstes Drehgelenk drehbar gelagert ist. Mit Hilfe einer ersten Antriebseinheit kann das Stativglied (4) bzw. die darauf angeordneten weiteren Komponenten des motorischen Stativs um die vertikale Achse A1 definiert gedreht werden. Die erste Antriebseinheit ist im dargestellten Ausführungsbeispiel in die vertikale Tragsäule (3) integriert und in dieser Darstellung nicht sichtbar. Am Stativglied (4) ist ein Gelenkparallelogramm über eine zweite Antriebseinheit (5) und ein zweites Drehgelenk angebracht. Mit dieser zweiten Antriebseinheit kann das Gelenkparallelogramm um eine horizontale Achse A2 relativ zur vertikalen Tragsäule (3) bzw. dem ersten Stativglied (4) positioniert werden. Das Gelenkparallelogramm, bestehend aus vier einzelnen Gelenkparallelogramm-Elementen (6, 7, 8, 9) sowie den entsprechenden weiteren Drehgelenken (10, 11, 12, 13), die diese Gelenkparallelogramm-Elemente (6, 7, 8, 9) drehbar verbinden, ist über eine dritte Antriebseinheit (14) um die Achse A3 bzw. dazu parallele Achsen durch die anderen Drehgelenke des Gelenkparallelogramms verstellbar. Die dritte Antriebseinheit (14) ist am Drehgelenk (13) des Gelenkparallelogramms angeordnet. Alternativ hierzu ist es möglich, die dritte Antriebseinheit zur Verstellung des Gelenkparallelogrammes an den anderen Drehgelenken (10, 11, 12) anzuordnen. Das Gelenkparallelogramm weist Ausbalancier-Gewichte (16, 17) auf, die z.B. an den beiden unteren Drehgelenken (10, 11) angeordnet werden können. Das Gelenkparallelogramm gewährleistet somit die Ausbalancierung um die horizontalen Achsen A2 und A3. Der in Verlängerung des oberen Gelenkparallelogramm-Elementes (9) angeordnete erste Tragarm (15) ist um seine Längsachse A4 drehbar beweglich und über eine vierte Antriebseinheit motorisch verstellbar. Hierzu ist der erste Tragarm (15) ebenfalls über ein Drehgelenk mit dem oberen Gelenkparallelogramm-Element (9) verbunden. Die vierte Antriebseinheit ist im dargestellten Ausführungsbeispiel in das obere Gelenkparallelogramm-Element (9) integriert und demzufolge in dieser Darstellung nicht sichtbar. Der erste Tragarm (15) ist konstruktiv vorzugsweise in doppelt-gekröpfter Bauweise ausgeführt, so daß für den jeweiligen Benutzer, der unter diesem Tragarm (15) steht, ein möglichst großer Bewegungsspielraum resultiert. Die Ausbalancierung bei Drehung um die Längsachse (14) wird ebenfalls durch die besondere konstruktive Gestaltung dieses ersten Tragarmes gewährleistet, was in Fig. 2 deutlich erkennbar ist. Am äußeren Ende des ersten Tragarmes (15) ist ein zweiter Tragarm (16) angeordnet, der mit einer fünften Antriebseinheit (17) um die Achse A5 bewegt werden kann. Mit dem ersten Tragarm (15) ist der zweite Tragarm über ein Drehgelenk verbunden. Der zweite Tragarm (16) weist an einem Ende ein Ausgleichsgewicht (19) auf, am anderen Ende ist ein dritter Tragarm (18) angeordnet, so daß bei Drehung um die Achse A5 eine Ausbalancierung um diese Achse A5 realisiert ist. Der dritte Tragarm (18) ist motorisch drehbar um seine Längsachse A6, was mit Hilfe einer sechsten Antriebseinheit erreicht werden kann, die nicht dargestellt wird und ebenfalls wieder über ein Drehgelenk mit dem zweiten Tragarm (16) verbunden ist. Es ist beispielsweise möglich, die sechste Antriebseinheit im Gehäuse des dritten Tragarmes (18) anzuordnen. An seinem freien Ende besitzt der dritte Tragarm (18) eine Schnittstelle für die verschiedensten medizinischen Therapie- oder Diagnoseinstrumente (20). In diesem Ausführungsbeispiel wird das verwendete Instrument lediglich schematisch dargestellt.

Die Achsen A4, A5, A6 sind im dargestellten Ausführungsbeispiel so im Raum orientiert, daß sie sich in einem Punkt schneiden und damit die sogenannte Roboterbedingung erfüllt ist.

Der Gesamtaufbau des erfindungsgemäßen motorischen Statives ist darauf ausgelegt, daß auch beim Ausfall der Antriebseinheiten ein mechanisch ausbalanciertes System zur Verfügung steht. Dies wird beispielsweise durch die Ausbalancierung mit Hilfe des Gelenkparallelogramms und den geeigneten Zusatzgewichten (10, 11) sowie dem Zusatzgewicht (19) am zweiten Tragarm (16) und die entsprechende konstruktive Gestaltung des ersten Tragarmes (15) erreicht. Durch die Abstimmung der Getriebereibungen in den eingesetzten sechs Antriebseinheiten ist jedoch zusätzlich gewährleistet, daß das Gewicht des jeweiligen Instrumentes in einem bestimmten Gewichtsintervall schwanken kann, ohne den motorischen oder manuellen Einsatz zu beeinträchtigen. Dabei müsssen die Getriebereibungen so ausgelegt werden, daß ein manuelles Bedienen des motorischen Statives noch möglich ist. Somit ist beispielsweise ein rascher Instrumentenwechsel während einer Operation möglich, ohne zeitaufwendige Ausbalancier-Prozeduren durchführen zu müssen.

Zum Betrieb des erfindungsgemäßen motorischen Statives ist weiterhin eine Steuereinheit (30) erforderlich, die die einzelnen Antriebseinheiten definiert ansteuert. Diese Steuereinheit (30) wird vorzugsweise über dem Fußteil (1) des erfindungsgemäßen motorischen Statives in einer geeigneten Konsole angeordnet. Um ein definiertes Positionieren des motorischen Statives zu gewährleisten, muß die Steuereinheit (30) laufend die aktuellen Raumkoordinaten des jeweiligen Instrumentes erfassen. Dies erfolgt über laufendes Auslesen der Winkelgeber, die mit jeder Antriebseinheit gekoppelt sind. Bei bekannten geometrischen Abmessungen des motorischen Statives bzw. der Einzelelemente und Stativglieder ist somit die Bestimmung der Instrumentenposition im Stativ-Koordinatensystem jederzeit möglich. Ist zudem der Zusammenhang zwischen Stativ-Koordinatensystem und Patienten-Koordinatensystem bekannt, so ist der stereotaktische Einsatz des erfindungsgemäßen motorischen Statives und des daran angeordneten Instrumentes gewährleistet.

Die Vorgabe bestimmter Zielkoordinaten für das jeweilige Therapie- oder Diagnoseinstrument durch den Chirurgen erfolgt über eine Eingabeschnittstelle, die mit der Steuereinheit gekoppelt ist. Als mögliche Eingabeschnittstellen kommen z.B. berührungsempfindliche Sensoren, optische Systeme, Joysticks oder aber Eingabe-Tastaturen in Frage. Dabei gibt die Steuereinheit für jede Antriebseinheit bestimmte Sollwerte vor, die angefahren werden. Über separate Regelkreise für die jeweiligen Antriebseinheiten werden diese Sollwerte anschließend definiert angefahren.

In Fig. 2 wird ein Ausführungsbeispiel des erfindungsgemäßen motorischen Statives perspektivisch dargestellt. Über dem runden Fußteil (101) ist eine Konsole (103) angeordnet, in der die Steuereinheit untergebracht ist. Die in Fig. 1 sichtbare vertikale Tragsäule und das erste Stativglied sind in dieser Darstellung demzufolge nicht erkennbar. Die Konsole (103) ist mit dem ersten Stativglied verbunden und dreht sich mit diesem Stativglied um die erste vertikale Achse A1. Über eine zweite Antriebseinheit (105) und ein Drehgelenk ist ein Gelenkparallelogramm am - nicht dargestellten - ersten Stativglied angeordnet. Das aus vier einzelnen Gelenkparallelogramm-Elementen (106, 107, 108, 109) bestehende Gelenkparallelogramm ist über die zweite Antriebseinheit (105) und das Drehgelenk um eine horizontale Achse A2 relativ zur Konsole (103) bzw. dem Fußteil (101) und dem ersten Stativglied und der vertikalen Tragsäule, die in dieser Darstellung nicht sichtbar sind, beweglich. Die vier einzelnen Gelenkparallelogramm-Elemente (106, 107, 108, 109) sind über in dieser Darstellung nicht sichtbare Drehgelenke in den Ecken des Gelenkparallelogramms relativ zueinander beweglich. Das untere Gelenkparallelogramm-Element (107) ist mit einem Ausgleichsgewicht zur Ausbalancierung versehen. Das Drehgelenk zwischen den Gelenkparallelogramm-Elementen (106) und (109) ist mit einer dritten Antriebseinheit (104) versehen, mit der eine motorische Relativ-Verstellung des Gelenkparallelogramms in sich möglich ist. Die Drehbewegung erfolgt dabei um eine weitere horizontale Achse A3, die durch das Drehgelenk geht. Auch hier ist eine andere Anordnung der dritten Antriebseinheit in einer der anderen Ecken des Gelenkparallelogrammes möglich. In Verlängerung des obersten Gelenkparallelogramm-Element (109) ist ein erster Tragarm (115) über ein Drehgelenk angeordnet, der um seine Längsachse A4 mittels einer vierten Antriebseinheit gedreht werden kann. Die Ausbalancierung bei Drehung um die Längsachse A4 wird durch die spezielle konstruktive Gestaltung des ersten Tragarmes (115) erreicht, was in dieser perspektivischen Darstellung verdeutlicht wird. Die hierzu erforderliche vierte Antriebseinheit ist in dieser Darstellung nicht erkennbar und kann beispielsweise im oberen Gelenkparallelogramm-Element (109) untergebracht werden. Deutlich erkennbar ist in dieser Darstellung der doppelt-gekröpfte Aufbau des ersten Tragarmes (115), der eine optimale Bewegungsfreiheit für den darunter operierenden Chirurgen zur Folge hat. Am äußeren Ende des ersten Tragarmes (115) ist über ein weiteres Drehgelenk ein zweiter Tragarm (116) inklusive einer fünften - nicht dargestellten - Antriebseinheit montiert. Mit Hilfe des Drehgelenkes und der fünften Antriebseinheit kann der zweite Tragarm (116) um eine weitere Achse A5 gedreht werden. Ein Ende des Tragarmes (116) ist mit Ausgleichsgewichten versehen, während am entgegengesetzten Ende ein dritter Tragarm (118) angeordnet ist. Dieser Tragarm (118) wiederum ist um seine Längsachse A6 über ein entsprechendes Drehgelenk und eine sechste - ebenfalls nicht dargestellte - Antriebseinheit motorisch bewegbar. Am Ende dieses Tragarmes (118) befindet sich eine Schnittstelle, an der in diesem Ausführungsbeispiel als medizinisches Therapie- oder Diagnoseinstrument ein Operationsmikroskop (120) befestigt ist.

Anhand von Fig. 3 wird im folgenden der Aufbau der jeweiligen Antriebseinheiten beschrieben, wobei der erfindungsgemäße Aufbau dieser Antriebseinheiten weitere Vorteile für den medizinischen Einsatz bietet. Sämtliche verwendeten Antriebseinheiten des erfindungsgemäßen motorischen Statives weisen diesen prinzipiellen Aufbau auf. Eine entsprechende Antriebseinheit umfaßt dabei den eigentlichen Antriebsmotor (41), beispielsweise einen Scheibenläufermotor, sowie ein Getriebe (42). Wie bereits erwähnt wird durch die geeignete Abstimmung der Getriebereibungen aller eingesetzten Antriebseinheiten eine zusätzliche mechanische Stabilisierung des gesamten Systemes erreicht. Geeignete Getriebe werden z.B. von der Fa. HARMONIC DRIVE unter der Bezeichnung HDGM vertrieben. Neben dem Spielraum für das Instrumentengewicht hat diese Dimensionierung der Getriebereibungen zur Folge, daß auch eine nicht exakt horizontale Aufstellung die Funktionsfähigkeit nicht beeinflußt. Des weiteren umfaßt jede Antriebseinheit eine Bremse (43), mit der eine Positionsfixierung jederzeit möglich ist. Hierzu kann z.B. eine Permanentmagnet-Einscheibenbremse verwendet werden. Unter sicherheitsrelevanten Aspekten ist weiterhin wichtig, daß jede Antriebseinheit zwei Winkelgeber (44a, 44b) umfaßt. Einer der beiden Winkelgeber (44a) ist hierbei direkt mit dem Antriebsmotor (41) gekoppelt, während der zweite Winkelgeber (44b) unabhängig vom Antriebsmotor (41) arbeitet. Im motorischen Betrieb gewährleistet der Einsatz zweier Winkelgeber eine gegenseitige Überwachung der erfaßten Winkeldaten. Desweiteren ist das motorische Stativ damit beim eventuellen Ausfall eines Winkelgebers weiterhin prinzipiell funktionsfähig. So ist auch beim Ausfall des Antriebsmotors (41) über den unabhängigen zweiten Winkelgeber (44b) eine Koordinatenerfassung prizipiell möglich. Zudem können eventuelle Getriebetoleranzen dadurch egalisiert werden. Im dargestellten Ausführungsbeispiel wird als mit dem Antriebsmotor (41) gekoppelter Winkelgeber (44a) ein herkömmlicher inkrementeller Winkelgeber verwendet, zu dessen Einsatz immer eine vorhergehende Referenzmessung erforderlich ist. Als zweiter - unabhängiger - Winkelgeber (44b) wird ein Code-Drehgeber eingesetzt, der laufend Absolut-Winkelmeßwerte der jeweiligen Gelenkbewegung liefert. Die Verwendung eines derartigen zweiten Winkelgebers bietet weiterhin den Vorteil, daß nach einem eventuellen Ausfall des ersten inkrementellen Winkelgebers kein zweites Referenzieren während der Operation erforderlich ist, sondern immer die aktuellen Winkelwerte direkt zur Verfügung stehen. Weiterhin ist auch bei einem Ausfall der Antriebseinheit jederzeit die Lageinformation gewährleistet. Hierzu geeignete Winkelgeber werden z.B. von der Firma HEIDENHAIN unter der Typenbezeichnung ROC 417 vertrieben. Ein Geschwindigkeitsgeber (45) sorgt des weiteren für die Kontrolle der Verfahrgeschwindigkeit des jeweiligen Elementes des erfindungsgemäßen motorischen Statives.

Sämtliche Elemente einer Antriebseinheit sind über entsprechende Signalleitungen mit der Steuereinheit verbunden, die sowohl die aktuellen Daten einliest und entsprechend auswertet, als auch die erforderlichen Steuersignale an die Antriebseinheit gibt. Dabei ist für jede Antriebseinheit ein separater Regelkreis vorgegeben, der das Anfahren bestimmter Sollwerte kontrolliert.

## Patentansprüche

1. Motorisches Stativ zur Positionierung medizinischer Diagnose- oder Therapieinstrumente, bestehend aus einem Mehrgelenkmechanismus mit integrierten Winkelgebern sowie Antriebseinheiten für die einzelnen Gelenke und einer Steuereinheit, dadurch gekennzeichnet, daß der Mehrgelenkmechanismus mechanisch ausbalanciert ist, um auch einen manuellen Betrieb des Statives zu ermöglichen, daß für jede Achse, um die eine Drehung möglich ist, eine Antriebseinheit vorgesehen ist, die jeweils einen Antriebsmotor (41) mit Getriebe (42), eine Bremse (43), Geschwindigkeitsgeber (45) sowie zwei Winkelgeber (44a, 44b) umfaßt und daß die Getriebereibungen der einzelnen Antriebseinheiten derart aufeinander abgestimmt sind, daß auch bei einer Variation des Instrumentengewichtes innerhalb eines definierten Gewichtsintervalls ein mechanisch ausbalanciertes Gesamtsystem resultiert.

2. Motorisches Stativ nach Anspruch 1, dadurch gekennzeichnet, daß zur Ausbalancierung Federn und/oder Ausgleichsgewichte vorgesehen sind.

3. Motorisches Stativ nach Anspruch 1, dadurch gekennzeichnet, daß für jede Antriebseinheit mindestens ein Regelkreis vorgesehen ist, der das Anfahren vorgegebener Sollwerte kontrolliert.

4. Motorisches Stativ nach Anspruch 1, dadurch gekennzeichnet, daß an einer vertikalen Tragsäule (3), ein Stativglied (4) angeordnet ist, das motorisch um eine vertikale Achse (A1) bewegbar ist, wobei an diesem Stativglied (4) ein motorisch um ein oder mehrere horizontale Achsen (A2, A3) verstellbares Gelenkparallelogramm mitsamt ein oder mehreren Ausgleichsgewichten (10, 11) angeordnet ist, an dem ein motorisch um eine weitere, vorzugsweise seine Längsachse (A4) bewegbarer erster Tragarm (15, 115) angeordnet ist, an dem ein zweiter Tragarm (16, 116) in Verlängerung angeordnet ist, der motorisch um eine zur Längsachse (A4) des ersten Tragarmes (15, 115) senkrechte weitere Achse (A5) bewegbar ist, wobei an einem freien Ende dieses zweiten Tragarmes (16, 116) ein dritter Tragarm (18, 118) angeordnet ist, der motorisch um seine Längsachse (A6) bewegbar ist und an dessen freien Ende eine Schnittstelle für ein medizinisches Therapie- oder Diagnoseinstrument angeordnet ist.

5. Motorisches Stativ nach Anspruch 4, dadurch gekennzeichnet, daß pro Antriebseinheit ein mit dem Antriebsmotor (41) gekoppelter inkrementeller Winkelgeber (44a) vorgesehen ist sowie ein vom Antriebsmotor (41) unabhängiger Winkelgeber (44b), der absolute Winkelwerte liefert.

6. Motorisches Stativ nach Anspruch 1, dadurch gekennzeichnet, daß die Steuereinheit (30) laufend die Winkelwerte der Winkelgeber (44a, 44b) erfaßt und daraus die Koordinaten des medizinischen Diagnose- oder Therapieinstrumentes im Stativ-Koordinatensystem ermittelt, wobei die Steuereinheit (30) desweiteren eine Eingabeschnittstelle zur definierten Vorgabe bestimmter Zielkoordinaten besitzt.

7. Motorisches Stativ nach Anspruch 6, dadurch gekennzeichnet, daß die Steuereinheit (30) über dem Fußteil (1, 101) des motorischen Statives angeordnet ist.

8. Motorisches Stativ nach Anspruch 1, dadurch gekennzeichnet, daß das motorische Stativ eine auf einem Fußteil (1) angeordnete, vertikale Tragsäule (3) umfaßt und das Fußteil motorisch ein- und ausfahrbare Rollen (2a, 2b, 2c) aufweist.

9. Motorisches Stativ nach Anspruch 4, dadurch gekennzeichnet, daß das Gelenkparallelogramm motorisch sowohl relativ zur vertikalen Tragsäule (3) oder dem Stativglied (4), an dem es angeordnet ist als auch motorisch in sich selbst um ein oder mehrere horizontale Achsen (A2, A5) verstellbar ist.

10. Motorisches Stativ nach Anspruch 4, dadurch gekennzeichnet, daß das Gelenkparallelogramm ein oder mehrere Ausgleichsgewichte (10, 11) umfaßt, die derart dimensioniert sind, daß das motorische Stativ bezüglich einer Bewegung um die horizontalen Achsen (A2, A3) ausbalanciert ist.

11. Motorisches Stativ nach Anspruch 4, dadurch gekennzeichnet, daß am freien Ende des zweiten Tragarmes (16,116) mindestens ein Ausgleichsgewicht angeordnet ist und der erste Tragarm derart konstruktiv gestaltet ist, daß das motorische Stativ bezüglich einer Bewegung um die Längsachse (A4) des ersten Tragarmes (15, 115) und/oder um die hierzu senkrechte Achse (A5), um die der zweite Tragarm (16, 116) drehbar ist, ausbalanciert ist.

12. Motorisches Stativ nach Anspruch 4, dadurch gekennzeichnet, daß der erste Tragarm (15, 115) in doppelt-gekröpfter Bauweise ausgeführt ist.

13. Motorisches Stativ nach mindestens einem der vorangehenden Ansprüche, dadurch gekennzeichnet, daß als medizinisches Therapie- oder Diagnoseinstrument ein Operationsmikroskop (20) vorgesehen ist.

14. Motorisches Stativ nach Anspruch 1, dadurch gekennzeichnet, daß die Antriebseinheiten mindestens einen Antriebsmotor (41) mit Getriebe (42), mindestens eine Bremse (43), mindestens einen Geschwindigkeitsgeber (45) sowie zwei Winkelgeber zum Erfassen der aktuellen Winkelmeßwerte umfassen, von denen mindestens einer absolute Winkelwerte liefert.

## Claims

1. Motorized stand for positioning medical diagnostic or therapeutic instruments, comprising a multi-jointed mechanism having integrated angle transducers and drive units for the individual joints and a control unit, characterized in that, the multi-jointed mechanism is mechanically balanced in order to enable a manual operation of the stand,
that a drive unit is provided for each axis about which driven rotation is arranged, and in that each of the drive units comprises a drive motor (41) with gears (42), a brake (43), a speed transducer (45), and two angle transducers (44a, 44b), and
that the individual drive units have gear frictions that are coordinated such that a mechanically balanced entire system results even when there is variation of the weight of the instrument within a defined range.

2. Motorized stand according to claim 1, characterized in that springs and/or balancing weights are provided for balancing.

3. Motorized stand according to claim 1, characterized in that at least one control circuit is provided for each of the drive units, the at least one control circuit being arranged for controlling approaching predetermined reference values.

4. Motorized stand according to claim 1, characterized in that a stand member (4) is arranged on a vertical support column (3) and is movable by a motor drive about a vertical axis (A1), a parallelogram linkage together with at least one balancing weight (10, 11) being arranged on said stand member (4) and being movable for displacement by a motor drive about at least one horizontal axis, a first support arm (15; 115) with a longitudinal axis (A4) being arranged on the parallelogram linkage and being movable by a motor drive about a further axis, which is preferably the longitudinal axis (A4) of the first support arm (15; 115), a second support arm (16; 116) being arranged on said first support arm (15; 115) as an extension of said first support arm (15; 115) and being movable by a motor drive about a still further axis (A5) perpendicular to the longitudinal axis (A4) of the first support arm (15; 115), a third support arm (18; 118) with a longitudinal axis (A6) and a free end being arranged at a free end of the second support arm (16; 116) and being movable by a motor drive about its longitudinal axis (A6), and having an interface for a medical diagnostic or therapeutic instrument being arranged at the free end of the third support arm (18; 118).

5. Motorized stand according to claim 4, characterized in that each of the drive units comprises an incremental angle transducer (44a) coupled to the drive motor of the drive unit and an angle transducer (44b) independent of the drive motor for supplying absolute angle values.

6. Motorized stand according to claim 1, characterized in that the control unit (30) continuously monitors the angle values of the angle transducers (44a, 44b) and determines from the angle values the coordinates of the medical diagnostic or therapeutic instrument in the coordinate system of the motorized stand, the control unit (30) having an input interface for defined provision of target coordinates.

7. Motorized stand according to claim 6, characterized in that the control unit (30) is arranged above the foot portion (1; 100) of the motorized stand.

8. Motorized stand according to claim 1, characterized in that the motorized stand comprises a vertical support column (3) arranged on a foot portion (1) and in that the foot portion has rollers (2a, 2b, 2c) and a motor drive for extending and retracing said rollers.

9. Motorized stand according to claim 4, characterized in that the parallelogram linkage is displaceable by a motor drive both relative to at least one of the vertical support column (3) and the stand member (4) on which it is arranged, and to itself about at least one of the horizontal axes (A2, A5).

10. Motorized stand according to claim 4, characterized in that the parallelogram linkage comprises at least one balancing weight (10, 11) having a weight such that the motorized stand is balanced in relation to motion about the horizontal axes (A2, A3).

11. Motorized stand according to claim 4, characterized in that at least one balancing weight is arranged at a further free end of the second support arm (16; 116) and in that the first support arm is constructionally formed such that the motorized stand is balanced in respect of motion about the longitudinal axis (A4) of the first support arm (15; 115) and/or about the axis (A5) perpendicular to the longitudinal axis (A4) of the first support arm (15; 115) about which axis (A5) the second support arm (16; 116) is rotatable.

12. Motorized stand according to claim 4, characterized in that the first support arm (15; 115) is embodied in a double cranked construction.

13. Motorized stand according to one of claims 1 to 12, characterized in that a surgical microscope (20) is provided as the medical diagnostic or therapeutic instrument.

14. Motorized stand according to claim 1, characterized in that the drive units comprise at least one drive motor (41) with gears (42), at least one brake (43), at least one speed transducer (45) and at least two angle transducers to monitor present angle measurement values, at least one of which angle transducers delivers absolute angle values.

## Revendications

1. Support motorisé servant à positionner des instruments médicaux de diagnostics ou de thérapie, comprenant un mécanisme polyarticulé à indicateurs d'angle intégrés, des unités motrices actionnant les diverses articulations et une unité de commande,
caractérisé en ce que
le mécanisme polyarticulé est équilibré mécaniquement, de manière qu'il soit possible de faire fonctionner le support à la main, en ce que
pour chaque axe autour duquel peut s'effectuer une rotation, il est prévu une unité motrice comprenant un moteur d'entraînement (41), une transmission (42), un frein (43), un indicateur de vitesse (45) et deux indicateurs d'angle (44a, 44b) et en ce que
les frottements de transmission des diverses unités motrices sont accordés entre eux de manière à obtenir, lorsque le poids des instruments varie, un équilibrage mécanique pondéral de l'ensemble du système, bénéficiant d'une marge au niveau du poids.

2. Support motorisé selon la revendication 1,
caractérisé en ce que
des ressorts et/ou des contrepoids d'équilibrage sont prévus pour assurer l'équilibrage.

3. Support motorisé selon la revendication 1,
caractérisé en ce qu'
il est prévu pour chaque unité motrice un circuit de régulation contrôlant que des valeurs théoriques données préalablement sont mises en route.

4. Support motorisé selon la revendication 1,
présentant les caractéristiques suivantes :
- sur une colonne porteuse verticale (3) est monté un élément de support (4) qu'un moteur peut faire tourner autour d'un axe vertical (A1),
- sur l'élément de support (4) est monté un parallélogramme articulé, équipé d'un ou plusieurs contrepoids d'équilibrage (10, 11), et mobile par l'action d'un moteur autour d'un ou de plusieurs axes (A2, A3),
- sur le parallélogramme articulé est monté un premier bras porteur (15, 115) mobile par l'action d'un moteur autour d'un autre axe, de préférence l'axe longitudinal (A4) du bras,
- le bras (15, 115) porte, dans son prolongement, un second bras porteur (16, 116) mobile par l'action d'un moteur autour d'un axe (A5) perpendiculaire à l'axe longitudinal (A4) du bras (15, 115),
- le second bras (16, 116) porte, à son extrémité libre, un troisième bras porteur (18, 118) pouvant tourner autour de son axe longitudinal (A6) par l'action d'un moteur, ce bras étant équipé, à son extrémité libre d'une interface de liaison à un instrument de thérapie ou de diagnostic.

5. Support motorisé selon la revendication 4,
caractérisé en ce que
il est prévu, pour chaque unité motrice, un indicateur d'angle (44a), incrémental, accouplé au moteur d'entraînement (41), ainsi qu'un indicateur d'angle (44b), indépendant du moteur (41) et délivrant les valeurs absolues de l'angle.

6. Support motorisé selon la revendication 1,
caractérisé en ce que
l'unité de commande (30) saisit en continu les valeurs angulaires données par les indicateurs (44a, 44b) et en déduit les coordonnées de l'instrument médical de diagnostic ou de thérapie, dans le système de coordonnées du support, l'unité de commande (30) possédant par ailleurs une interface pour allouer des coordonnées définies à atteindre.

7. Support motorisé selon la revendication 6,
caractérisé en ce que
l'unité motrice (30) est montée sur l'embase (1, 101) du support motorisé.

8. Support motorisé selon la revendication 1,
caractérisé en ce que
le support motorisé comprend une colonne porteuse (3) montée sur une embase (1) équipée de roulettes (2a, 2b, 2c) pouvant sortir ou rentrer par l'action d'un moteur.

9. Support motorisé selon la revendication 4,
caractérisé en ce que
le parallélogramme articulé peut se déplacer par l'action d'un moteur, par rapport à la colonne porteuse (3) et par rapport à l'élément de support (4) sur lequel il est monté, le parallélogramme peut également, par l'action d'un moteur, se régler lui-même en tournant autour d'un ou plusieurs axes horizontaux (A2, A5).

10. Support motorisé selon la revendication 4,
caractérisé en ce que
le parallélogramme articulé comporte un ou plusieurs contrepoids d'équilibrage (10, 11) dimensionnées de manière que le support motorisé est équilibré en ce qui concerne un mouvement autour des axes horizontaux (A2, A3).

11. Support motorisé selon la revendication 4,
caractérisé en ce que
le second bras porteur (16, 116) est équipé, à son extrémité libre d'au moins un contrepoids tandis que le premier bras porteur est construit de manière que le support motorisé est équilibré par rapport à un mouvement autour de l'axe longitudinal (A4) du premier bras porteur (15, 115) et/ou autour de l'axe (A5), perpendiculaire au précédent et autour duquel peut tourner le second bras porteur (16, 116) .

12. Support motorisé selon la revendication 4,
caractérisé en ce que
le premier bras porteur (15, 115) est doublement coudé.

13. Support motorisé selon au moins une des revendications précédentes,
caractérisé en ce que
il est prévu comme instrument médical de thérapie ou de diagnostic, un microscope d'opération.

14. Support motorisé selon la revendication 1,
caractérisé en ce que
les unités motrices comprennent chacune au moins un moteur d'entraînement (41) équipé d'une transmission, au moins un frein (43), au moins un indicateur de vitesse (45) ainsi que deux indicateurs d'angle saisissant les valeurs instantanées de l'angle et dont au moins l'un d'eux délivre des valeurs d'angle absolues.
